# EUROPÄISCHE PATENTSCHRIFT

(11) **EP 0 373 111 B1**
(45) Veröffentlichungstag und Bekanntmachung des Hinweises auf die Patenterteilung: **17.03.1993**
(21) Anmeldenummer: 89810900.4
(22) Anmeldetag: 23.11.1989
(51) Int. Cl.: A61C 8/00, A61C 3/02, A61B 17/16

(54) **Einrichtung zur Behandlung eines Knochens, insbesondere Kieferknochens, und/oder Zahns**
Device for treating a bone, especially a jaw bone and/or a tooth
Dispositif pour le traitement d'un os, en particulier de la mâchoire, et/ou des dents

(30) Priorität: 02.12.1988 CH 4486/88
(43) Veröffentlichungstag der Anmeldung: 13.06.1990
(73) Patentinhaber: INSTITUT STRAUMANN AG, CH-4437 Waldenburg (CH)
(72) Erfinder: Sutter, Franz, CH-4435 Niederdorf (CH); Frei, Alexander, CH-4718 Holderbank (CH); Schwammberger, Andreas Eduard, CH-4411 Seltisberg (CH)
(74) Vertreter: Eder, Carl E.

(56) Entgegenhaltungen:
- CH-A- 657 807
- DE-A- 2 948 682
- DE-A- 2 949 556
- DE-A- 3 433 570

## Beschreibung

Die Erfindung betrifft eine Einrichtung gemäss dem Oberbegriff des Anspruchs 1. Die Einrichtung ist zur Behandlung eines Knochens, insbesondere Kieferknochens, und/oder Zahns vorgesehen und vor allem dazu bestimmt, um einen Kieferknochen mit mindestens einem Loch zu versehen, in welchem ein Implantat für die Befestigung eines künstlichen Zahnersatzes verankert werden kann.

Implantate für den besagten Zweck sind zum Beispiel aus der US-A-4 180 910, der US-A-4 447 209 sowie dem Buch "Orale Implantologie", André Schroeder, Franz Sutter und Gisbert Krekeler, Georg Thieme Verlag Stuttgart, New York, 1988, Titelseite und Seiten 66 - 71, 118 - 151, 178 - 187, 202 - 217, 228 - 243 bekannt. Diese Implantate besitzen einen zum Einsetzen in einen Kieferknochen bestimmten Sockel mit mindestens einem hohlzylindrischen Teil. Zum Verankern eines Implantats in einem Kieferknochen wird dieser für den bzw. jeden hohlzylindrischen Teil des Sockels mit einem Loch versehen. Dieses kann abhängig von der Art und den Abmessungen des Implantats beispielsweise einen durch eine volle, d.h. über ihren ganzen Durchmesser hohle Bohrung gebildeten Mündungsabschnitt und einen durch eine Ringnut gebildeten Grundabschnitt aufweisen oder ausschliesslich aus einer vollen Bohrung oder ausschliesslich aus einer Ringnut bestehen und eventuell auch noch mit einem Innengewinde versehen sein. Im genannten Buch sind auch Werkzeuge zum Herstellen von Löchern in Kieferknochen offenbart. Damit beim Herstellen der Löcher das diese begrenzende Knochenmaterial nicht abstirbt, sollte es nicht über eine ca. 45°C bis 47°C betragende Maximal temperatur erwärmt werden. Wie es ebenfalls im genannten Buch erwähnt ist, können die Werkzeuge zur Vermeidung einer übermässigen Erwärmung mit einem flüssigen Kühlmittel, etwa einer wässerigen, sterilen, physiologischen Kochsalz- oder Ringer-Lösung gekühlt werden, die gemäss dem Buch zum Beispiel auf der Aussenseite der Werkzeuge zugeführt werden kann.

Eine aus der DE-C-3 433 570 bekannte Einrichtung weist ein drehbares Werkzeug mit zwei lösbar mit einander verschraubten Stücken auf. Das eine von diesen bildet einen Schneidteil sowie einen mit einem Innengewinde versehenen Ansatz und das andere den Schaft oder - genauer gesagt - mindestens dessen Hauptteil. Das den Schaft-Hauptteil bildende Stück des Werkzeuges hat ein mit dem erwähnten Innengewinde verschraubtes Aussengewinde, einen Bund und auf dessen dem Schneidteil abgewandter Seite eine Ringnut. Das Werkzeug hat einen dessen Innen-Kühlung mit einem flüssigen Kühlmittel ermöglichenden Durchgang. Dieser besitzt ein axiales sich von der Stirnseite am freien Ende des Schneidteils bis mindestens zur Ringnut erstreckendes Loch und eine dieses mit der Ringnut verbindende Radialbohrung. Eine zum Zuführen des Kühlmittels zum Werkzeug dienende, den Schaft im Bereich der Ringnut umschliessende Hülse ist zwischen dem Bund des Schafts und einem separaten, auf dem Schaft befestigten Stellring drehbar sowie axial unverschiebbar gehalten. Die Hülse ist mit je einem Dichtungsring gegen den Bund sowie den Stellring abgedichtet, begrenzt zusammen mit der Ringnut des Schafts einen Ringkanal und ist mit einem in diesen mündenden Kühlmittel-Einlass versehen.

Da die mit einander verschraubten Gewinde der beiden Stücke des Werkzeuges eine gewisse Mindestlänge besitzen müssen und da auch der Stellring eine gewisse axiale Ausdehnung hat, wird das Werkzeug zwangsläufig verhältnismässig lang und insbesondere länger als ein Werkzeug, das einen gleichartigen Schneidteil besitzt, der konventionell, d.h. nicht zur Innen-Kühlung ausgebildet ist. Die verhältnismässig grosse Länge des aus der DE-C-3 433 570 bekannten Werkzeuges erschwert die mittels des Werkzeuges erfolgenden, zahnärztlichen Behandlungen, und zwar besonders die im Bereich der Mahlzähne stattfindenden Behandlungen. In der DE-C-3 433 570 ist nicht beschrieben, wie der Stellring befestigt ist. Bei im allgemeinen gemäss dieser Publikation ausgebildeten, auf dem Markt bekannten Einrichtungen ist der Stellring auf dem Schaft aufgepresst, d.h. durch eine Press-Sitz-Verbindung befestigt, so dass es für einen Zahnarzt und dessen Personal praktisch nicht möglich ist, die Hülse vom Schaft zu entfernen und wieder auf diesem zu montieren. Das Werkzeug und die Hülse müssen daher vor dem Gebrauch in zusammengebautem Zustand gereinigt sowie steril gemacht werden, was eine einwandfreie Reinigung und Sterilisation erschwert. Zudem ist die Fabrikation der Einrichtung wegen der grossen Anzahl herzustellender und mit einander zu verbindenen Teile verhältnismässig aufwendig.

Der Erfindung liegt daher die Aufgabe zugrunde, eine Einrichtung zu schaffen, die die Nachteile der aus der DE-C-3 433 570 bekannten Einrichtung und der im allgemeinen gemäss dieser Publikation ausgebildeten, auf dem Markt bekannten Einrichtungen behebt und insbesondere ermöglicht, die Hülse ohne einen Stellring oder sonstige separate, in axialer Richtung Platz beanspruchende Verbindungsmittel sowie vorzugsweise leicht entfernbar mit dem Werkzeug zu verbinden, wobei die Einrichtung wirtschaftlich herstellbar sein soll.

Diese Aufgabe wird durch eine Einrichtung gelöst, die erfindungsgemäss die Merkmale des Anspruchs 1 aufweist. Vorteilhafte Ausgestaltungen der Einrichtung gehen aus den abhängigen Ansprüchen hervor.

Der Erfindungsgegenstand wird anschliessend anhand in der Zeichnung dargestellter Ausführungsbeispiele erläutert. In der Zeichnung zeigt
die Figur 1 eine vereinfachte Schrägansicht einer Einrichtung zum Behandeln eines Kieferknochens,
die Figur 2 eine Schrägansicht des Werkzeuges und der zum Einleiten eines Kühlmittels in dieses dienenden Hülse der in der Figur 1 dargestellten Einrichtung in grösserem Massstab,
die Figur 3 einen Axialschnitt durch das Werkzeug und die Hülse,
die Figur 4 eine Draufsicht auf die sich in der Figur 2 unten befindende Stirnseite des den Schaft des Hohlfräsers bildenden Werkstückes,
die Figur 5 den die Verrastung des Werkzeuges mit der Hülse darstellenden Ausschnitt V aus der Figur 3 in grösserem Massstab,
die Figur 6 eine Schrägansicht eines anderen Werkzeuges und einer Hülse,
die Figur 7 eine Schrägansicht noch eines anderen Werkzeuges und einer Hülse,
die Figur 8 einen Schnitt durch ein Stück eines Kieferknochens mit einem Loch und
die Figur 9 einen Axialschnitt durch einen Abschnitt eines Werkzeuges und einer Hülse mit anderen Rast-Mitteln.

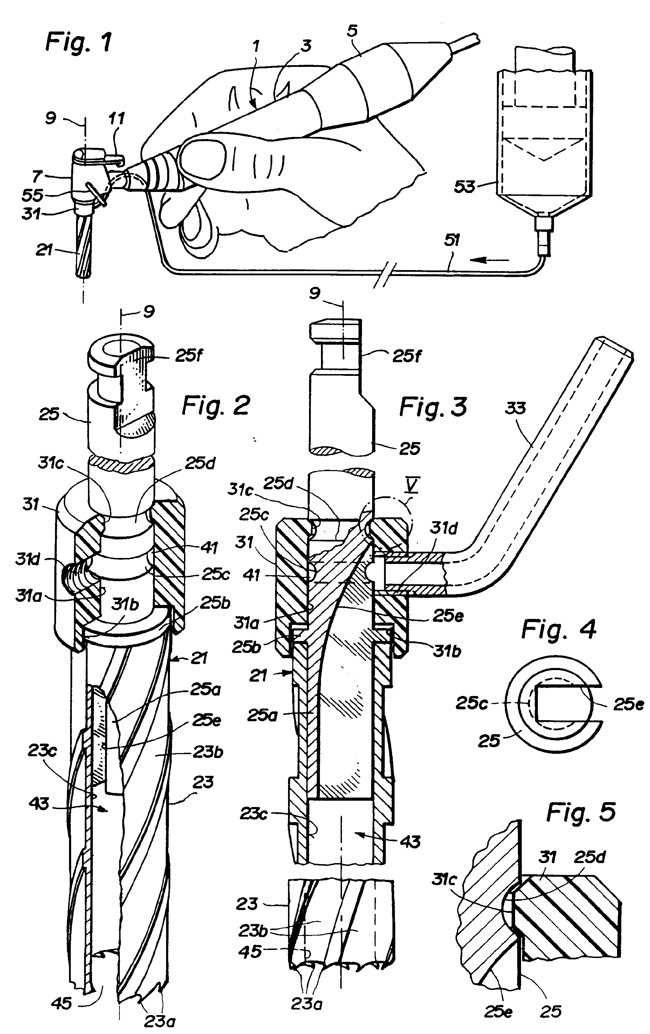

Die in der Figur 1 ersichtliche Einrichtung zur Behandlung eines Kieferknochens weist eine Antriebsvorrichtung 1 mit einem Handstück 3 auf, das mit einem elektrischen oder pneumatischen Motor 5, einem Winkelstück 7 und einem mehrteiligen Gehäuse versehen ist. Im zum Winkelstück 7 gehörenden Gehäuse oder Gehäuseteil ist ein Winkelgetriebe mit einem um eine Achse 9 drehbaren Abtriebselement angeordnet. Das Winkelstück 7 weist ferner Verschluss- und/oder Kupplungsmittel mit einem manuell verschwenkbaren Betätigungshebel 11 auf. Ein um die Achse 9 drehbares Werkzeug 21 ist durch die Verschluss-und/oder Kupplungsmittel lösbar und starr mit dem Abtriebselement verbunden, d.h. gekuppelt, wobei die Verbindung oder Verkupplung durch Verschwenken des Betätigungshebels 11 verriegelt und wieder entriegelt werden kann.

Das in den Figuren 2 sowie 3 in von der Antriebsvorrichtung 1 getrenntem Zustand ersichtliche Werkzeug 21 ist im allgemeinen zur Achse 9 rotationssymmetrisch und besitzt einen Schneidteil 23 sowie einen Schaft 25. Bei dem als Hohlstirnfräser ausgebildeten Werkzeug 21 sind der Schneidteil 23 sowie der Schaft 25 je aus einem ursprünglich separaten Körper, d.h. Werkstück gebildet und durch Verschweissen starr, unlösbar und dicht miteinander verbunden. Der Schneidteil 23 besteht aus einer im allgemeinen zylindrischen Hülse und hat an seinem freien Ende mehrere, nämlich acht über seinen Umfang verteilte Schneiden 23a sowie auf seiner Mantel-Aussenseite mehrere, nämlich vier entlang von Schraubenlinien verlaufende Spannuten 23b und im zentralen Querschnittsbereich ein axiales, durchgehendes Loch 23c. Das den Schaft 25 bildende Werkstück hat an seinem schneidteilseitigen Ende einen in das Loch 23c hinein ragenden Endabschnitt 25a und eine diesen vom restlichen Hauptteil des Schafts abgrenzende, radial nach aussen ragende ringförmige, einen Bund bildende Verdickung 25b, die an der den Schneiden abgewandten End-Ringfläche des Schneidteils 23 anliegt. Der auf der dem Schneidteil 23 abgewandten Seite an die Verdickung 25b abschliessende Hauptteil des Schafts 25 hat zwei die Achse 9 ringförmig umschliessende, im Axialschnitt bogenförmige Nuten, von denen die sich näher bei der Verdickung 25b befindende mit 25c bezeichnet ist und die andere als Rast-Nut 25d dient. Der Schaft 25 ist ferner mit einem sich in axialer Richtung erstreckenden Einschnitt 25e versehen. Dieser verläuft von einer sich auf der dem Schneidteil 23 abgewandten Seite der Nut 25c und etwa zwischen dieser und der Rast-Nut 25d und/oder in der Nähe des schneidteilseitigen Randes der letzteren befindenden Anfangsstelle bis zur Stirnfläche des sich im Loch 23c des Schneidteils 23 befindenden Ende des Schafts 25. Der Grund des Einschnitts 25e ist mindestens in dessen dem Schneidteil 23 abgewandten Anfangsabschnitt derart zur Längsrichtung des Werkzeuges geneigt, dass seine radial gemessene Tiefe zum freien, mit den Schneiden 23a versehenen Ende des Schneidteils 23 hin zunimmt. Der Grund des Einschnitts 25e hat nämlich einen im Axialschnitt konkav gebogenen Abschnitt, welcher bei der besagten Anfangsstelle des Einschnitts auf der Mantel- oder Umfangsfläche des Schafts beginnt. An den gebogenen Abschnitt des Einschnittgrundes schliesst stetig ein zur Achse 9 paralleler Abschnitt an, der sich bis zum im Loch 23c des Schneidteils 23 angeordneten Ende des den Schaft bildenden Werkstücks erstreckt. Im Querschnitt ist der Einschnitt 25e gemäss der Figur 4 rechteckförmig. An seinem dem Schneidteil 23 abgewandten Ende ist der Schaft mit einem Kupplungsabschnitt 25f versehen, der zur drehfesten Verbindung des Werkzeuges 21 mit dem Abtriebselement des Winkelstückes 7 eine zur Achse 9 parallele Abflachung und eine bogenförmige, beidenends in diese mündende, zur axialen Verriegelung des Werkzeuges im Winkelstück dienende Nut aufweist.

Auf dem sich zwischen dem Schneidteil 23 und dem Kupplungsabschnitt 25f befindenden Schaft-Längsabschnitt ist eine Hülse 31 mit einem axialen, durchgehenden Loch 31a gelagert. Dieses hat am schneidteilseitigen Ende eine Erweiterung 31b, welche die Verdickung 25b und damit auch die von dieser auf der dem Schneidteil 23 abgewandten Seite gebildete Schulter sowie zudem noch das den Schneiden 23a abgewandte Schneidteil-Ende aufnimmt. Die das Loch 31a begrenzende Innenfläche der Hülse 31 ist in der Nähe von deren dem Schneidteil 23 abgewandten Ende mit einer ringförmigen, radial zur Achse 9 hin ragenden Rippe versehen, die einen Rast-Vorsprung 31c bildet und eine Verengung des Lochs 31a begrenzt. Wie es besonders deutlich in der Figur 5 ersichtlich ist, ragt der Rast-Vorsprung 31c in die Rast-Nut 25d hinein und hat geneigte, sich in Richtung zum Nutgrund hin an einander annähernde Flanken. Der Schaft 25 und die Hülse 31 sind derart ausgebildet, dass sie bezüglich einander mit kleinem, radialem Spiel gut und leicht, d. h. mit nur geringer Reibung um die Achse 9 drehbar sind. Die Rast-Nut 25d und der Rast-Vorsprung 31c bilden mit dem übrigen Teil des Schafts 25 bzw. der Hülse 31 zusammen je aus einem einstückigen Körper bestehende Rast-Mittel 25d, 31c, welche die Hülse 31 bezüglich des Schafts 25 sowohl gegen vom Schneidteil 23 weg als auch gegen zu diesen hin gerichtete, axiale Verschiebungen sichern und also die Hülse - abgesehen von einem allenfalls vorhandenen, kleinen axialen Spiel - axial unverschiebbar, aber lösbar am Werkzeug halten. Der Mantel der Hülse 31 ist im Bereich der Nut 25c des Schafts 25 mit einem von aussen her in die axiale Öffnung 31a mündenden Kühlmittel-Einlass 31d, nämlich einer radialen Gewindebohrung versehen. In diese ist ein als Anschluss 33 dienendes, abgewinkeltes oder abgebogenes Rohrstück eingeschraubt, das einen bezüglich der Achse 9 vom radialen Einlass 31d und vom Schneidteil 23 weg geneigten Abschnitt hat.

Der Kühlmittel-Einlass 31d mündet in einen von der Hülse 31 und der Nut 25c des Schafts 25 begrenzten, ringförmigen, den Schaft 25 umschliessenden Kanal 41. Dieser ist fluidmässig mit einem vom Einschnitt 25e des Schafts 25 und vom freien Teil der Öffnung 23c des Schneidteils 23 gebildeten Durchgang 43 verbunden, der in im allgemeinen axialer Richtung durch einen Teil des Werkzeuges hindurch verläuft und am freien Ende des Schneidteils mit einer vom Ende des Lochs 31a gebildeten Mündungsöffnung 45 in die Umgebung mündet. Der Anschluss 33 ist durch einen auf ihn aufgesteckten, als Kühlmittel-Zuleitung 51 dienenden, flexiblen Schlauch mit dem Ausgang einer Kühlmittel-Zufuhrvorrichtung 53 verbunden, die aus einer Spritze mit einem im allgemeinen zylindrischen Behälter und einem über einen Schaft manuell in diesen hinein drückbaren Kolben besteht. Der Anschluss 33 und/oder die Zuleitung 51 kann noch mit einem Halter 55 lösbar am Winkelstück 7 gehalten sein. Der Halter 55 kann beispielsweise durch einen etwa U- oder C-förmigen, lösbar am Gehäuse des Winkelstückes angeklipsten Bügel gebildet sein.

Das Werkzeug 21 besteht aus einem metallischen Material, etwa Stahl. Die Hülse 31 besteht aus einem thermoplastischen Kunststoff. Dieser soll derart beschaffen sein, dass die Hülse 31 formfest, aber ausreichend elastisch deformierbar ist, um ein Ein- und Ausrasten der Rast-Mittel 25d, 31c zu ermöglichen. Des weiteren soll die das axiale Loch 31a begrenzende Innenfläche der Hülse gute Gleitungseigenschaften haben. Damit die Hülse 31 durch beispielsweise mit heissem Wasser und/oder heissem Dampf stattfindendes Erhitzen steril gemacht werden kann, soll der sich bildende Kunststoff ferner bis zu mindestens 120°C und vorzugsweise bis zu mindestens 180°C oder und beispielsweise zumindest kurzfristig bis zu 200°C oder sogar bis zu mindestens 220°C betragenden Temperaturen hitzebeständig sein. Zudem soll die Hülse gleich wie übrigens auch das Werkzeug 21 aus einem biokompabiblen, ungiftigen Material bestehen. Schliesslich soll der zur Bildung dienende Kunststoff gut verarbeitbar und insbesondere extrudierbar und/oder spritzgiessbar sein. Ein für die Bildung der Hülse 31 geeigneter, die genannten Anforderungen erfüllender Kunststoff ist beispielsweise Polyäther-Ätherketon (PEEK). Der Anschluss 33 kann beispielsweise aus einem metallischen Material bestehen.

Da die zum Zuführen des flüssigen Kühlmittels zum Werkzeug 21 und zum Einleiten des Kühlmittels in den Durchgang 43 des Werkzeuges 21 dienende Hülse 31 die Verdickung 25b des Schafts 25 und sogar noch einen kleinen Abschnitt des Schneidteils 23 übergreift, befindet sie sich unmittelbar hinter dem für eine Knochen-Behandlung nutzbaren Abschnitt des Schneidteils 23. Das zum Einstecken des Werkzeuges 21 dienende Ende des Abtriebselements des Winkelstückes 7 hat eine ringförmige, radiale Endfläche und ragt beispielsweise ein wenig aus dem Gehäuse des Winkelstückes 7 heraus und/oder ist ungefähr mit dem Gehäuse bündig. Wenn das Werkzeug 21 gemäss der Figur 1 mit dem Winkelstück 7 verbunden ist, steckt der Schaft 25 in einem axialen Loch des Abtriebselements. Die Hülse 31 befindet sich dann zumindest annähernd unmittelbar vor der Öffnung des Winkelstück-Gehäuses, bei welcher das Abtriebselement sowie der Schaft 25 aus diesem Gehäuse austreten. Dabei ist die Hülse 31 durch einen schmalen, ringspaltförmigen Zwischenraum von der besagten Endfläche des beim Betrieb rotierenden Abtriebselements und/oder vom Gehäuse des Winkelstückes getrennt. Der Abstand zwischen der schneidteilseitigen Begrenzung des Winkelstückes 7 und dem winkelstückseitigen Ende des für eine Kieferknochen-Behandlung nutzbaren Abschnitts des Schneidteils 23 braucht also nur ganz geringfügig grösser zu sein als die in axialer Richtung gemessene Abmessung der Hülse 31. Diese Abmessung der Hülse 31 beträgt höchstens das dreifache und beispielsweise ungefähr das zweifache des Durchmessers des zylindrischen Hauptteils des Schafts 25. Der Aussendurchmesser des Schaft-Hauptteils des für die Kieferknochen-Behandlung dienenden Werkzeuges 21 kann beispielsweise etwa 2,35 mm betragen. Die axiale Abmessung der Hülse 31 kann dann höchstens 7 mm und beispielsweise ungefähr 5 mm betragen. Die Länge des Werkzeuges 21 und damit auch die parallel zur Achse 9 gemessene Gesamtabmessung des Winkelstückes 7 und Werkzeuges 21 kann daher verhältnismässig kurz bemessen werden, was insbesondere bei der Behandlung eines Kieferknochens im Bereich der Mahlzähne vorteilhaft ist.

Der ringförmige, von der Nut 25c des Schafts 25 sowie der Hülse 31 begrenzte Kanal 41 ist zunächst durch die beidseitig von ihm paarweise mit geringem radialem Spiel auf einander aufliegenden zylindrischen Flächenabschnitte der Mantelfläche des Schafts 25 und der Innenfläche der Hülse 31 gegen die Umgebung der Hülse 31 abgeschlossen. Auf der dem Schneidteil 23 zugewandten Seite des Kanals 41 ergeben zudem die von der Verdickung 25b im Inneren der Erweiterung 31b des Lochs 31a gebildete Schulter und der die zylindrische Umfangsfläche der Verdickung 25b mit höchstens kleinem, radialem Spiel umschliessende Randabschnitt der Hülse 31 zusammen eine im Axialschnitt etwa z-förmige Labyrinthdichtung. Auf der dem Schneidteil abgewandten Seite des ringförmigen Kanals 41 wird dieser zusätzlich durch den in die Rast-Nut 25d hineinragenden, aus einer ringförmigen Rippe bestehenden, den Schaft vollständig sowie unterbruchslos umschliessenden Rast-Vorsprung 31c abgedichtet. Der Kanal 41 wird also - abgesehen vom in den Kühlmittel-Einlass 31 eingeschraubten Anschluss 33 - ausschliesslich durch Abschnitte des Schafts 25 des Werkzeuges 21 und der Hülse 31 mehr oder weniger flüssigkeitsdicht gegen die Umgebung abgeschlossen, ohne dass hiezu separate, gummielastische Dichtungsringe vorhanden sind.

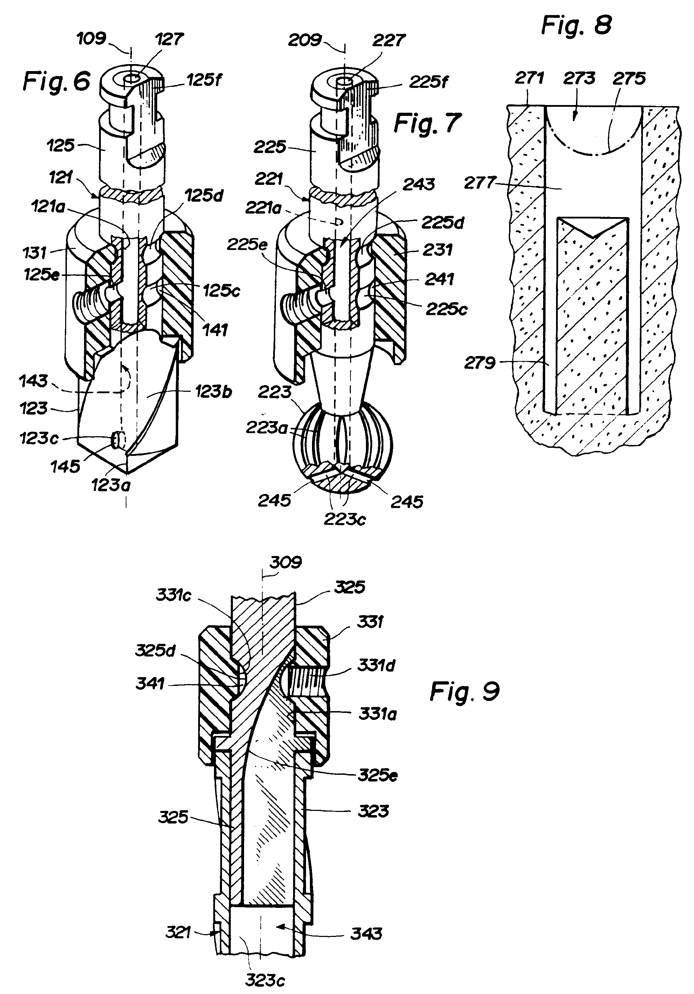

Die Figur 6 zeigt ein als Spiralbohrer ausgebildetes, um eine Achse 109 drehbares Werkzeug 121 mit einem Schneidteil 123 und einem Schaft 125, wobei der Schneidteil und der Schaft bei diesem Werkzeug aus einem zusammenhängenden, einstückigen Körper bestehen. Das Werkzeug 121 ist mit einem sich von der Endfläche des freien Schaftendes bis in die Nähe des freien Endes des Schneidteils 123 erstreckenden, koaxialen Loch 121a, nämlich einer zylindrischen Sackbohrung versehen. Das Loch 121a ist am schaftseitigen Ende durch ein in seinen Endabschnitt eingepresstes und/oder eingeschweisstes, zylinderförmiges Verschlusselement 127 dicht abgeschlossen. Der Schneidteil 123 hat an seinem freien Ende zwei Schneiden 123 oder - genauer gesagt - Hauptschneiden und im Bereich seiner Umfangsfläche zwei Spannuten 123b. Der Schneidteil 123 besitzt ferner zwei mit der Achse 109 einen Winkel bildende, d. h. radiale oder zur Achse 109 geneigte Löcher 123c, die in der Nähe des freien Endes des Schneidteils 123 vom Endabschnitt des axialen Loches 121a je in eine der beiden Spannuten 123b münden. Der Durchmesser des Schneidteils 123 oder - genauer gesagt - des Schneidteil-Hüllkreiszylinders ist grösser als der Durchmesser des Schafts 125, so dass das schaftseitige Ende des Schneidteils 123 zusammen mit dem Schaft 125 mindestens in den nicht von den Spannuten eingenomenen Umfangsbereichen analog wie die Verdickung 25b des Schafts 25 eine Schulter mit einer radialen Fläche bildet. Der Schaft 125 hat eine ringförmige Nut 125c und eine ringförmige Rast-Nut 125d, die ananlog angeordnet und ausgebildet sind wie die Nuten 25c bzw. 25d des Schafts 25. Der Schaft 125 ist ferner mit einem von der Nut 125c in das axiale Loch 121a mündenden Loch 125e, nämlich einer radialen Bohrung versehen. An seinem dem Schneidteil 123 abgewandten Ende besitzt der Schaft 125 einen analog wie der Kupplungsabschnitt 25f ausgebildeten Kupplungsabschnitt 125f.

Eine Hülse 131 ist identisch wie die Hülse 31 ausgebildet oder sogar durch diese selbst gebildet und am Schaft 125 analog wie die Hülse 31 am Schaft 25 gehalten. Der schneidteilseitige Hülsenrandabschnitt übergreift den an den Schaft 125 anschliessenden, bezüglich diesem eine Verdickung des Werkzeuges 121 bildenden Endabschnitt des Schneidteils 123 ähnlich wie der entsprechende Randabschnitt der Hülse 31 die Verdickung 25b des Schafts 25 und einem kurzen Abschnitt des Schneidteils 23. Im übrigen ist die Hülse 131 mit einem nicht gezeichneten, dem Anschluss 33 entsprechenden Anschluss versehen. Die Hülse 131 begrenzt zusammen mit der Nut 125c einen ringförmigen Kanal 141. Der freie Teil des axialen Lochs 121a bildet zusammen mit dem radialen Loch 125e und den Löchern 123c einen Durchgang 143, der von den äusseren Enden der beiden Löcher 123c gebildete, im Bereich des Schneidteils in die Umgebung mündende Mündungsöffnungen 145 hat.

Das in der Figur 7 ersichtliche, um eine Achse 209 drehbare Werkzeug 221 hat einen kugelförmigen oder, genauer gesagt, kugelabschnittförmigen Schneidteil 223 und einen beispielsweise zusammen mit diesem aus einem einstückigen Körper bestehenden Schaft 225. Das Werkzeug 221 hat ein zur Achse 209 koaxiales Loch 221a, nämlich eine sich vom freien Ende des Schafts 225 bis in die Nähe des schneidteilseitigen Werkzeugendes erstreckende, beim freien Schaftende durch ein Verschlusselement 227 abgeschlossene Sackbohrung. Der Schneidteil 221 weist mehrere über seinen Umfang verteilte Schneiden 223a und mit der Achse 209 einen Winkel bildende und nämlich von dieser sowie vom Schaft weg geneigte Löcher 223c auf. Diese verlaufen vom schneidteilseitigen Ende des axialen Lochs 221a in den dem Schaft abgewandten, halbkugelförmigen Teil der Mantelfläche des Schneidteils 223. Der Schaft 225 hat einen zylindrischen Hauptteil, der an seinen schneidteilseitigen Enden durch einen konischen Hals mit dem Schneidteil verbunden ist. Der Schaft 225 ist mit einer Nut 225c und einer Rast-Nut 225d versehen, die analog wie die Nuten 25c bzw. 25d angeordnet und ausgebildet sind. Der Schaft 225 hat ferner ein die Nut 225c mit dem axialen Loch 221 verbindendes, radiales Loch 225e und einen Kupplungsabschnitt 225f.

Eine gleich wie die Hülse 31 beschaffene oder durch diese selbst gebildete Hülse 331 ist drehbar und axial unverschiebbar, lösbar auf dem Schaft 225 gehalten und begrenzt zusammen mit dessen Nut 225c einen ringförmigen Kanal 241. Der freie Teil des axialen Lochs 221a bildet zusammen mit dem radialen Loch 225e des Schafts 225 und den zur Achse 209 geneigten Löchern 223c des Schneidteils 223 einen Durchgang 243, der bei den von den äusseren Enden der Löcher 223c gebildeten Mündungsöffnungen 245 in die Umgebung mündet.

Die Hülsen 131 und 231 werden auf den Schäften 125 bzw. 225 in analoger Weise drehbar und axial unverschiebbar gehalten, wie es für die Hülsen 31 und den Schaft 25 beschrieben wurde. Die Schäfte der drei Werkzeuge 21, 121, 221 haben zudem identisch ausgebildete Kupplungsabschnitte 25f, 125f, 225f, so dass wahlweise eines der drei Werkzeuge in das Winkelstück 7 gesteckt und über dessen Winkelgetriebe durch den Motor der Antriebsvorrichtung 1 gedreht werden kann. Die Schneidteile der drei Werkzeuge 21, 121, 221 haben beispielsweise mindestens ungefähr gleiche Aussendurchmesser. Die Einrichtung mit der Antriebsvorrichtung 1 und den drei Werkzeugen 21, 121, 221 kann verwendet werden, um den Knochen 271, nämlich Kieferknochen 271, von dem in der Figur 8 ein Ausschnitt ersichtlich ist, ein Loch 273 zum Aufnehmen eines Implantats der in der Einleitung beschriebenen Art zu versehen. Dazu wird zuerst das Werkzeug 221 in das Winkelstück 7 eingesetzt und im Knochen ein in der Figur 8 strichpunktiert angedeutetes Markierungsloch 275 angebracht. Danach wird das Werkzeug 121 in das Winkelstück 7 eingesetzt und mit dem Werkzeug 121 beim Markierungsloch 275 eine Sackbohrung in den Knochen gebohrt, die den zylindrischen, über den ganzen Durchmesser hohlen Mündungs-Abschnitt 277 des Lochs 273 bildet. Schliesslich wird mit dem Werkzeug 21 ausgehend vom kegelförmigen Grund der Sackbohrung der ringnutförmige Grundabschnitt 279 des Lochs 273 in den Knochen gefräst.

Nun sollen noch einige Betriebseigenschaften der Einrichtung erörtert werden, wobei als Beispiel die Behandlung des Kieferknochens mit dem Werkzeug 21 näher erläutert wird. Dieses wird beim Behandeln des Knochens durch den Motor der Antriebsvorrichtung 1 über das Winkelgetriebe des Winkelstückes - in der Figur 1 von oben her gesehen - im Uhrzeigersinn gedreht. Wenn sich die Hülse 31 frei drehbar auf dem Schaft 25 des Werkzeuges 21 befindet, ist dieses bestrebt, die Hülse 31 bis in diejenige Stellung mitzudrehen, in welcher der vom Schneidteil 23 weg nach oben geneigte Abschnitt des Anschlusses 33 und/oder der auf diesem aufgesteckte, die Zuleitung 33 bildende Schlauch an der sich in der Figur 1 hinten befindenden Seite des Gehäuses des Winkelstückes 7 anliegt. Zudem wird der Anschluss 33 und/oder die Zuleitung 33 durch den Halter 55 am Winkelstück gehalten. Die Hülse 31 wird daher durch den Anschluss 33 sowie die Zuleitung 51 in Zusammenwirkung mit dem Halter 55 bezüglich des Gehäuses der Antriebs vorrichtung undrehbar festgehalten, so dass sie also vom Werkzeug nicht mitgedreht wird. Die Tatsache, dass zwischen dem Schaft 25 und der Hülse 31 keine separaten, gummielastischen Dichtungsringe vorhanden sind, trägt dazu bei, dass das Werkzeug 21 und die Hülse 31 gut und leicht, d.h. mit geringer Reibung bezüglich einander drehbar sind. Beim zum Fräsen des Loch-Grundabschnitts 279 stattfindenden Drehen des Werkzeuges kann beispielsweise eine Hilfsperson des die Behandlung durchführenden Zahnarztes oder Chirurgen die Zufuhrvorrichtung 53 betätigen, d.h. deren Kolben in den Behälter hinein drücken und dadurch ein im Behälter der Zufuhrvorrichtung gespeichertes, flüssiges Kühlmittel, nämlich beispielsweise eine wässerige Lösung mit einer ungefähr 5°C betragenden Temperatur durch die Zuleitung 51, den Anschluss 33 sowie die Hülse 31 zum Werkzeug 21 zu führen und in dessen Durchgang 43 einleiten. Das Kühlmittel kann dann durch den Durchgang 43 zur Mündungsöffnung 45 strömen, bei dieser in die Umgebung austreten und den Schneidteil 23 sowie inbesondere dessen Schneiden 23a und auch den behandelten Knochen im Bereich der Behandlungsstelle kühlen. Wie weiter vorne beschrieben, schliessen der Schaft 25 und die Hülse 31 den von ihnen begrenzten, ringförmigen Kanal 41 zumindest annähernd flüssigkeitsdicht gegen die Umgebung ab. Allenfalls beim schneidteilseitigen Rand der Hülse 31 doch noch zwischen dieser und der Werkzeug-Umfangsfläche austretendes Kühlmittel wird durch den die Verdickung 25b übergreifenden Randabschnitt der Hülse 31 zum freien Ende des Schneidteils 23 hin geleitet, so dass dieses Kühlmittel ebenfalls noch zur Kühlung des Werkzeuges und behandelten Knochenbereichs beitragen.

Bei den Werkzeugen 121 und 221 werden die von deren Nuten 125c bzw. 225c und den Hülsen 131 bzw. 231 begrenzten, ringförmigen Kanäle 141 bzw. 241 in ähnlicher Weise wie beim Werkzeug 21 und der Hülse 31 durch Abschnitte der Werkzeuge und Hülsen mindestens annähernd flüssigkeitsdicht gegen die Umgebung abgeschlossen. Beim Werkzeug 121, bei welchem der Schneidteil 123 mit kleinem radialem Spiel in die der Erweiterung 31b entsprechende Erweiterung des axialen Lochs der Hülse 131 hinein ragt und sich die Spannuten 123b bis in den schneidteilseitigen Endabschnitt der Hülse 121 erstrecken, wird allenfalls beim schneidteilseitigen Ende der Hülse zwischen dieser und dem Werkzeug austretendes Kühlmittel vor allem in die Spannuten hinein geleitet, wo es ebenfalls zur Kühlung des Schneidteils beiträgt.

Man kann jedes Werkzeug 21, 121, 221 - wie in den Figuren 1 bis 3 sowie 5 bis 7 dargestellt - mit einer ihm zugeordneten und lösbar auf seinem Schaft gehaltenen Hülse 31 bzw. 131 bzw. 231 ausrüsten. Wenn das für eine bestimmte Behandlungsphase benötigte Werkzeug in das Winkelstück 7 eingesetzt wird, kann man dann jeweils den die Zuleitung 51 bildenden Schlauch auf den Anschluss der betreffenden Hülse stecken. Es besteht jedoch auch die Möglichkeit, ein und dieselbe Hülse, zum Beispiel die Hülse 31 für die Zufuhr des Kühlmittels zu den drei Werkzeugen 21, 121, 221 zu verwenden. In diesem Fall kann man beim Wechseln der Werkzeuge jeweils die Hülse 31 vom Schaft des vorher benutzten Werkzeuges wegnehmen und auf den Schaft des zu benutzenden Werkzeuges stecken. Das Entfernen der Hülse vom sie haltenden Werkzeug-Schaft kann mühelos und schnell dadurch geschehen, dass man die Hülse mit einer Hand oder einem Instrument fasst und eine vom Schneidteil weggerichte Kraft auf sie ausübt. Dadurch kann zuerst der Rast-Vorsprung 31c der Hülse aus der Rast-Nut des betreffenden Werkzeuges ausgerastet und danach die Hülse zum freien Ende des Schafts verschoben sowie von diesem weggenommen werden. Anschliessend kann die Hülse ebenso mühelos und schnell auf das freie Schaftende des zu benutzenden Werkzeuges gesteckt und entlang dem Schaft in Richtung zum Schneidteil hin geschoben werden, bis die Rast-Mittel einrasten.

Wie aus der Figur 8 ersichtlich ist, umschliesst der ringnutförmige Grundabschnitt 279 des Lochs 273 einen mit dem restlichen Knochen zusammenhängenden, aus Knochenmaterial bestehenden Knochenzapfen, der nachher in den Innenraum des hohlzylindrischen Sockelteils des nicht gezeichneten Implantats hineinragt. In der Praxis kann es jedoch beim Fräsen des ringnutförmigen Loch-Grundabschnitts 279 gelegentlich geschehen, dass der Knochenzapfen abbricht und im Loch 23c des Schneidteils 23 des Werkzeuges 21 steckenbleibt. Im einem solchen Fall kann man das Werkzeug 21 aus dem Loch des Knochens herausziehen und vom Winkelstück trennen. Danach kann man die Hülse 31 vom Schaft 25 des Werkzeuges wegnehmen, einen länglichen, beispielsweise etwas biegbaren, aber zur Übertragung von Druckkräften geeigneten Teil eines Ausstoss-Instruments in den Einschnitt 25e einführen und den abgebrochenen, im Schneidteil 23 steckenden Knochenzapfen in vom Schaft 25 weg verlaufende Richtung aus dem Schneidteil herausstossen. Anschliessend kann die Hülse 31 nötigenfalls wieder auf den Schaft 25 gesteckt und das Loch im Knochen fertig gestellt werden.

Die Werkzeuge und Hülsen sind nach ihrer Benützung und vor ihrer Verwendung für die Behandlung eines anderen Patienten jeweils zu reinigen und zu sterilisieren. Die Hülsen können für die Reinigung von den Werkzeugen entfernt werden, wodurch eine einwandfreie Reinigung wesentlich erleichtert wird. Die Sterilisierung der Werkzeuge und Hülsen kann beispielsweise mit heissem Wasser und/oder Wasserdampf bei einer mindestens etwa 120°C und beispielsweise etwa 180°C bis 220°C betragenden Temperatur erfolgen, wobei die Hülsen bei der Sterilisation auf den Werkzeugen montiert oder von diesen getrennt sein können.

Das Winkelstück 7 ist vorzugsweise noch mit einer in bekannter Weise an der Aussenseite seines Gehäuses befestigten, nicht gezeichneten Düse versehen, die eine gegen die Umfangsfläche des Werkzeug-Schneidteils gerichtete Austrittsmündung und einen Anschluss zum Zuführen eines Kühl mittels hat. Diese Düse ermöglicht, die Werkzeuge 21, 121, 221 zusätzlich zur Innenkühlung durch ein ihren Durchgängen 43, 143, 243 zugeführtes Kühlmittel oder stattdessen auf ihren Aussenseiten zu kühlen. Wenn die Werkzeuge 21, 121, 221 benutzt werden, ohne ihren Durchgängen 43, bzw. 143 bzw. 243 ein Kühlmittel zuzuführen, kann man die Hülsen von den Werkzeugen entfernen oder zumindest darauf verzichten, die Anschlüsse der Hülsen mit der Zuleitung 51 zu verbinden.

Die Figur 9 zeigt einen Teil eines um eine Achse 309 drehbaren Werkzeuges 321 mit einem Schneidteil 323 und einem Schaft 325, auf dem eine Hülse 331 lösbar gehalten ist. Der Schneidteil 323 ist beispielsweise ähnlich oder gleich wie der Schneidteil 23 ausgebildet und hat ein axiales Loch 323c. Der Schaft 325 hat einem dem Einschnitt 25e entsprechenden Einschnitt 325e. Dieser erstreckt sich vom sich im Loch 323c befindenden Ende des Schafts 325 bis in den von der Hülse 331 umschlossenen Schaftabschnitt und zwar mindestens bis zur Rast-Nut 325d. Die Hülse 331 hat einen aus einer ringförmigen Rippe bestehenden, in die Rast-Nut 325d hinein ragenden Rast-Vorsprung 331c und einen im Bereich des Rast-Vorsprungs in das axiale Loch 331a der Hülse mündenden Kühlmittel-Einlass 331d, der durch eine radiale Gewindebohrung gebildet ist, in die ein nicht gezeichneter, gleich oder ähnlich wie der Anschluss 33 ausgebildeter Anschluss eingeschraubt werden kann. Die Querschnittsformen und -abmessungen der Rast-Nut 325d und des Rast-Vorsprung 331c sind derart bemessen, dass zwischen dem Nutgrund und dem Rast-Vorsprung ein ringförmiger Kanal 341 frei bleibt. Dieser ist mit dem vom Loch 323c und dem Einschnitt 325e gebildeten Durchgang 343 des Werkzeuges 321 verbunden. Das Werkzeug 321 unterscheidet sich also vom Werkzeug 21 dadurch, dass anstelle der beiden ringförmigen Nuten 25c, 25d nur die Rast-Nut 325d vorhanden ist, die sowohl zur lösbaren Verrastung der Hülse 331 als auch zur Bildung des Kanals 341 dient. Abgesehen von diesem Unterschied und der zur Anpassung an diesen dienenden Änderung der Hülse sind das Werkzeug 321 und die Hülse 331 ähnlich oder gleich ausgebildet wie das Werkzeug 21 bzw. die Hülse 31.

Die Einrichtungen können noch auf andere Arten geändert werden. Man kann insbesondere Merkmale der verschiedenen Werkzeuge und Hülsen miteinander kombinieren. Beispielsweise kann man selbstverständlich auch bei den Werkzeugen 121, 221 die beiden Nuten 125c, 125d bzw. 225c, 225d durch eine einzige Nut ersetzen, die analog wie die Rast-Nut 325d sowohl zum Verrasten der Hülse auch auch zur Begrenzung eines ringförmigen Kanals dient. Die Hülsen 131, 231 wären dann dementsprechend gleich oder ähnlich wie die Hülsen 331 auszubilden. Des weiteren könnte man auch die Schäfte des Werkzeuges 121 und 221 mit einer entsprechend der Verdickung 25b des Schafts 25 ausgebildeten und angeordneten Verdickung versehen, die von der der Erweiterung 31b entsprechenden Erweiterung des axialen Lochs der auf den betreffenden Schaft gehaltenen Hülse aufgenommen werden kann. Es wäre jedoch auch möglich, den die Erweiterung 31b der Hülse 31 begrenzenden Kragen von dieser wegzulassen, so dass sich die Hülse 31 in den Figuren 2, 3 nur bis zur oberen, ringörmigen Radialfläche der Verdickung 25b erstreckt. Entsprechendes gilt für die Hülsen der anderen Ausführungsbeispiele. Zudem könnte man die Einschnitte 25e, 325e der Schäfte 25 bzw. 325 durch ein sich vom schneidteilseitigen Schaftende bis zum ringförmigen Kanal 41 bzw. 341 ersteckendes, axiales Loch und ein radiales, dieses mit der Nut 25c bzw. der Rast-Nut 225d verbindendes Loch ersetzen.

Die Rast-Mittel könnten eventuell derart ausgebildet werden, dass sie die Hülsen lediglich gegen vom Schneidteil des betreffenden Werkzeuges weggerichtete Verschiebungen sichern und Verschiebungen in der Gegenseite zulassen. Zum Einschneidteil hin gerichtete Verschiebungen der Hülsen könnten dann durch mindestens eine von der Achse weg nach aussen ragende Schulter- und/oder Anschlagfläche des Werkzeuges verhindert werden, wie sei beim Werkzeug 21 von der Verdickung 25b und beim Werkzeug 121 von dessen an den Schaft anschliessenden Schneidteil gebildet werden.

Die Formen und Abmessungen der Werkzeuge können selbstverständlich entsprechend von deren Zwecken in manigfaltiger Weise geändert werden. Bei den Werkzeugen 21, 321 bei denen der Schneidteil und der Schaft durch ursprünglich getrennte Werkstücke gebildet sind, könnten die beiden Werkstücke statt durch Schweissverbindungen durch Löt- oder Klebverbindungen starr und unlösbar miteinander verbunden werden. Zudem können die Werkzeuge auch zur Behandlung von Zähnen ausgebildet werden, so dass dann mit dem Kühlmittel zusätzlich zum Schneidteil eines Werkzeuges auch der mit diesem behandelte Zahnbereich gekühlt werden kann. Des weiteren können die Einrichtungen und ihre Werkzeuge auch vorgesehen werden, um statt Unter- oder Oberkieferknochen irgendwelche andere Knochen zu behandeln und insbesondere mit Löchern zu versehen.

Der freie Hohlraum der ringförmigen, die Achsen und Schäfte der Werkzeuge umschliessenden Kanäle könnten statt durch eine in der Mantelfläche des betreffenden Schafts angebrachte Nut oder zusätzlich dazu durch eine in der Innenfläche der Hülse angebrachte, ringförmige Nut gebildet werden.

Die verschiedenen Werkzeuge und Hülsen können kostengünstig hergestellt werden. Des weiteren können die Hülsen rasch und einfach auf den Werkzeugen montiert und von diesen getrennt werden. Dabei wirkt es sich insbesondere günstig auf die Herstellungs- und Montagekosten aus, dass keine durch separate Werkstücke gebildete Befestigungsteile und Dichtungen erforderlich sind, um die Hülsen auf den Werkzeugen gegen axiale Verschiebungen zu sichern und abzudichten.

## Patentansprüche

1. Einrichtung zur Behandlung eines Knochens (271), insbesondere Kieferknochens (271), und/oder Zahns, mit einem um eine Achse (9, 109, 209, 309) drehbaren, einen Schneidteil (23, 123, 223, 323) sowie einen Schaft (25, 125, 225, 325) aufweisenden Werkzeug (21, 121, 221, 321) und einer auf dem Schaft (25, 125, 225, 325) angeordneten, mit diesem zusammen einen ringförmigen Kanal (41, 141, 241, 341) begrenzenden und einen in diesen mündenden Kühlmittel-Einlass (31d, 331d) aufweisenden Hülse (31, 131, 231, 331), wobei das Werkzeug (21, 121, 221, 321) sowie die Hülse (31, 131, 231, 331) relativ zu einander drehbar sowie gegen axiale Verschiebungen gesichert sind und wobei das Werkzeug (21, 121, 221, 321) einen vom Kanal (41, 141, 241, 341) zum Schneidteil (23, 123, 223, 323) verlaufenden Durchgang (43, 143, 243, 343) aufweist, dadurch gekennzeichnet, dass die Hülse (31, 131, 231, 331) mindestens gegen vom Schneidteil (23, 123, 223, 323) weg gerichtete Verschiebungen durch am Schaft (25, 125, 225, 325) und an ihr vorhandene, mit einander verrastete Rast-Mittel (25d, 31c, 125d, 225d, 325d, 331c) gesichert ist.

2. Einrichtung nach Anspruch 1, dadurch gekennzeichnet, dass die Rast-Mittel (25d, 31c, 125d, 225d, 325d, 331c) mit dem übrigen Teil des Schafts (25, 125, 225, 325) bzw. dem übrigen Teil der Hülse (31, 131, 231, 331) zusammen aus einem einstückigen Körper bestehen und durch eine bezüglich des Werkzeuges (21, 121, 221, 321) auf die Hülse (31, 131, 231, 331) ausgeübte, vom Schneidteil (23, 123, 223, 323) weggerichtete Kraft ausrastbar sind und dass der Schaft (25, 125, 225, 325) derart ausgebildet ist, dass die Hülse (31, 131, 231, 331) nach dem Ausrasten durch vom Schneidteil (23, 123, 223, 323) weggerichtetes Verschieben vom Werkzeug (21, 121, 221, 321) entfernbar ist.

3. Einrichtung nach Anspruch 1 oder 2, dadurch gekennzeichnet, dass die Rast-Mittel (25d, 31c, 125d, 225d, 325d, 331c) die Hülse (31, 131, 231, 331) auch gegen zum Schneidteil (23, 123, 223, 323) hin gerichtete Verschiebungen sichern.

4. Einrichtung nach einem der Ansprüche 1 bis 3, dadurch gekennzeichnet, dass die Rast-Mittel (25d, 31c, 125d, 225d, 325d, 331c) durch eine in der Umfangsfläche des Schafts (25, 125, 225, 325) vorhandene, ringförmige Rast-Nut (25d, 125d, 225d, 325d) und mindestens einen in diese eingreifenden Rast-Vorsprung (31c, 331c) der Hülse (31, 131, 231, 331) gebildet sind.

5. Einrichtung nach Anspruch 4, dadurch gekennzeichnet, dass der Rast-Vorsprung (31c, 331c) durch eine ringförmige, den Schaft (25, 125, 225, 325) vollständig sowie unterbruchslos umschliessenden Rippe gebildet ist.

6. Einrichtung nach Anspruch 4 oder 5, dadurch gekennzeichnet, dass sich die Rast-Nut (25d, 125d, 225d) und der Rast-Vorsprung (31c) auf der dem Schneidteil (23, 123, 223) abgewandten Seite des Kanals (41, 141, 241) befinden.

7. Einrichtung nach Anspruch 4 oder 5, dadurch gekennzeichnet, dass die Rast-Nut (325d) einen Teil der Begrenzung des Kanals (341) bildet.

8. Einrichtung nach einem der Ansprüche 1 bis 7, dadurch gekennzeichnet, dass der ringförmige Kanal (41, 141, 241, 341) sowohl auf seiner dem Schneidteil (23, 123, 223, 323) zugewandten als auch auf seiner diesem abgewandten Seite ausschliesslich durch Abschnitte des Werkzeuges (21, 121, 221, 321) und der Hülse (31, 131, 231, 331) gegen die Umgebung des Werkzeuges (21, 121, 221, 321) und der Hülse (31, 131, 231, 331) abgeschlossen ist, wobei die Hülse (31, 131, 231, 331) beispielsweise ein axiales, durchgehendes, am schneidteilseitigen Ende mit einer Erweiterung (31b) versehenes, vom Werkzeug (21, 121, 321) durchdrungenes Loch (31a) und das Werkzeug (21, 121, 321) beispielsweise auf der dem Schneidteil (23, 123, 323) zugewandten Seite des ringförmigen Kanals (41, 141, 341) eine von einer Verdickung (25b) des Schafts (25) und/oder vom Schneidteil (23, 123) gebildete, sich im Innern der Erweiterung (31b) befindende Schulter hat.

9. Einrichtung nach einem der Ansprüche 1 bis 8, dadurch gekennzeichnet, dass der die Hülse (31, 131, 231, 331) haltende Schaft (25, 125, 225, 325) starr und unlösbar mit dem Schneidteil (23, 123, 223, 323) verbunden ist und beispielsweise mit diesem verschweisst oder zusammen mit diesem aus einem einstückigen Körper gebildet ist, wobei die Hülse (31, 131, 231, 331) zweckmässigerweise aus einem elastisch deformierbaren Kunststoff besteht, der vorzugsweise spritzgiessbar und vorzugsweise bis zu einer mindestens 120°C und beispielsweise mindestens 180°C betragenden Temperatur hitzebeständig ist.

10. Einrichtung nach einem der Ansprüche 1 bis 9, wobei der Durchgang (43, 343) ein axiales Loch (23c, 323c) mit einer in das freie Ende des Schneidteils (23, 323) mündenden Mündungsöffnung (45) aufweist, dadurch gekennzeichnet, dass der Schaft (25, 325) einen den ringförmigen Kanal (41, 141) mit dem axialen Loch (23c, 323c) des Durchganges (41, 341) verbindenden, axial verlaufenden Einschnitt (25e, 325e) aufweist, dessen Grund mindestens in einem Teilbereich derart zur Längsrichtung des Werkzeuges (21, 321) geneigt ist, dass seine radial gemessene Tiefe zum freien Ende des Schneidteils hin zunimmt, und dass der Einschnitt (25e, 325e) durch die Hülse (31, 331) und/oder den Schneidteil (23, 123) gegen die Umgebung abgedeckt ist, wobei der Schneidteil (23, 323) und der Schaft (25, 325) vorzugsweise aus ursprünglich getrennten Werkstücken gebildet sind, das den Schaft (25, 325) bildende Werkstück vorzugsweise in das den Schneidteil (23, 323) bildende Werkstück hinein ragt und der Einschnitt (25e, 225e) sich vorzugsweise bis zum schneidteilseitigen Ende des den Schaft (25, 225) bildenden Werkstückes erstreckt.

## Claims

1. Device for the treatment of a bone (271), especially a jaw bone (271), and/or a tooth, comprising a tool (21, 121, 221, 321), which is rotatable about an axis (9, 109, 209, 309) and has a cutting part (23, 123, 223, 323) as well as a shaft (25, 125, 225, 325), and a sleeve (31, 131, 231, 331), which is arranged on the shaft (25, 131, 231, 331), defines together with the latter an annular duct (41, 141, 241, 341) and has a coolant inlet (31d, 331d) opening into this, wherein the tool (21, 121, 221, 321) as well as the sleeve (31, 131, 231, 331) are rotatable relative to one another as well as secured again axial displacements and wherein the tool (21, 121, 221, 321) has a passage (43, 143, 234, 343) extending from the duct (41, 141, 241, 341) to the cutting part (23, 123, 223, 323), characterised thereby that the sleeve (31, 131, 231, 331) is secured at least against displacements directed away from the cutting part (23, 123, 223, 323) by interengaging catch means (25d, 31c, 125d, 225d, 325d, 331e) present at the shaft (25, 125, 225, 325) and at the sleeve.

2. Device according to claim 1, characterised thereby that the catch means (25d, 31c, 125d, 225d, 325d, 331e) together with the remaining part of the shaft (25, 125, 225, 325) or the remaining part of the sleeve (31, 131, 231, 331) consist of a one-piece body and are disengageable by a force exerted in direction away from the cutting part (23, 123, 223, 323) and on the sleeve (31, 131, 231, 331) with respect to the tool (21, 121, 221, 321) and that the shaft (25, 125, 225, 325) is constructed in such a manner that after the disengaging the sleeve (31, 131, 231, 331) is removable from the tool (21, 121, 221, 321) by displacement directed away from the cutting part (23, 123, 223, 323).

3. Device according to claim 1 or 2, characterised thereby that the catch means (25d, 31c, 125d, 225d, 325d, 331e) also secure the sleeve 31, 131, 231, 331) against displacements towards the cutting part (23, 123, 223, 323).

4. Device according to one of claims 1 to 3, characterised thereby that the catch means (25d, 31c, 125d, 225d, 325d, 331e) are formed by an annular catch groove (25d, 125d, 225d, 325d) present in the circumferential surface of the shaft (25, 125, 225, 325) and at least one catch projection (31c, 331c), which is engageable in the groove, of the sleeve (31, 131, 231, 331).

5. Device according to claim 4, characterised thereby that the catch projection (31c, 331c) is formed by an annular rib which completely as well as uninterruptedly surrounds the shaft (25, 125, 225, 325).

6. Device according to claim 4 or 5, characterised thereby that the catch groove (25d, 125d, 225d) and the catch projection (31c) are disposed on the side of the duct (41, 141, 241) remote from the cutting part (23, 123, 223).

7. Device according to claim 4 or 5, characterised thereby that the catch groove (325d) forms a part of the boundary of the duct (341).

8. Device according to one of claims 1 to 7, characterised thereby that the annular duct (41, 141, 241, 341) is closed relative to the surroundings of the tool (21, 121, 221, 321) and of the sleeve (31, 131, 231, 331) not only at its side facing the cutting part (23, 123, 223, 323) but also at its side remote therefrom exclusively by portions of the tool (21, 121, 221, 321) and of the sleeve (31, 131, 231, 331), wherein the sleeve (31, 131, 231, 331) has, for example, an axial, continuous hole (31a), which is penetrated by the tool (21, 121, 321) and provided with an enlargement (31b) at the end at the side of the cutting part, and the tool (21, 121, 321) has, for example, at the side of the annular duct (41, 141, 341) facing towards the cutting part (23, 123, 323) a shoulder which is formed by a thickened portion (25b) of the shaft (25) and/or by the cutting tool (23, 123) and is disposed in the interior of the enlargement (31b).

9. Device according to one of claims 1 to 8, characterised thereby that the shaft (25, 125, 225, 325) holding the sleeve (31, 131, 231, 331) is rigidly and non-detachably connected with the cutting part (23, 123, 223, 323) and for example is welded to this or is formed with this from a one-piece body, wherein the sleeve (31, 131, 231, 331) expediently consists of an elastically deformable plastics material, which is preferably injection mouldable and preferably heat resistant up to a temperature amounting to at least 120°C and for example at least 180°C.

10. Device according to one of claims 1 to 9, wherein the passage (43, 343) has an axial hole (23c, 323c) with an outlet opening (45) opening into the free end of the cutting part (23, 323), characterised thereby that the shaft (25, 325) has an axially extending groove (25e, 325e), which connects the annular duct (41, 141) with the axial hole (23c, 323c) of the passage (41, 341) and the base of which at least in a part area is so inclined relative to the longitudinal direction of the tool (21, 321) that its radially measured depth increases towards the free end of the cutting part, and that the groove (25e, 325e) is covered over relative to the surroundings by the sleeve (31, 331) and/or the cutting part (23, 123), wherein the cutting part (23, 323) and the shaft (25, 325) are preferably formed from originally separate workpieces, the workpiece forming the shaft (25, 325) preferably projects into the workpiece forming the cutting part (23, 323) and the groove (25e, 225e) preferably extends up to the end, which is at the side of the cutting part, of the workpiece forming the shaft (25, 225).

## Revendications

1. Dispositif de traitement d'un os (271), en particulier d'un os maxillaire (271), et/ou d'une dent, comportant un outil (21, 221, 321) capable de tourner autour d'un axe (9, 109, 209, 309), muni d'une partie coupante (23, 123, 223, 333) et d'une queue (25, 125, 225, 325) et un manchon (31, 131, 231, 331) disposé sur la queue (25, 125, 225, 325), délimitant avec celle-ci un canal annulaire (41, 141, 241, 341) et présentant une entrée de réfrigérant (31d, 331d) débouchant dans celui-ci, l'outil (21, 121, 221, 321) et le manchon (31, 131, 231, 331) pouvant tourner l'un par rapport à l'autre et étant empêchés de se déplacer axialement, et l'outil (21, 121, 221, 321) présentant un passage (43, 143, 243, 343) qui s'étend du canal (41, 141, 241, 341) à la partie coupante (23, 123, 223, 323), caractérisé en ce que le manchon (31, 131, 231, 331) est protégé au moins contre les déplacements de direction opposée à la partie coupante (23, 123, 223, 323) par des dispositifs de verrouillage (25d, 31c, 125d, 225d, 325d, 331c) prévus sur la queue (25, 125, 225, 325) et sur le manchon et verrouillés entre eux.

2. Dispositif selon la revendication 1, caractérisé en ce que les dispositifs de verrouillage (25d, 31c, 125d, 225d, 325d, 331c) consistent avec la partie restante de la queue (25, 125, 225, 325) ou avec la partie restante du manchon (31, 131, 231, 331) en un corps d'une pièce et sont déverrouillables par une force exercée, par rapport à l'outil (21, 121, 221, 321), sur le manchon (31, 131, 231, 331), de direction opposée à la partie coupante (23, 123, 223, 323) et en ce que la queue (25, 125, 225, 325) est agencée de telle manière que le manchon (31, 131, 231, 331) peut être retiré de l'outil (21, 121, 221, 321) après le déverrouillage, par un déplacement de direction opposée à la partie coupante (23, 123, 223, 323).

3. Dispositif selon la revendication 1 ou 2, caractérisé en ce que les dispositifs de verrouillage (25d, 31c, 125d, 225d, 325d, 331c) protègent également le manchon (31, 131, 231, 331) contre les déplacements dirigés vers la partie coupante (23, 123, 223, 323).

4. Dispositif selon l'une des revendications 1 à 3, caractérisé en ce que les dispositifs de verrouillage (25d, 31c, 125d, 225d, 325d, 331c) sont formés par une rainure de verrouillage annulaire (25d, 125d, 225d, 325d) prévue dans la surface périphérique de la queue (25, 125, 225, 325) et par au moins une saillie de verrouillage (31c, 331c) prévue sur le manchon (31, 131, 231, 331), qui pénètre dans celle-ci.

5. Dispositif selon la revendication 4, caractérisé en ce que la saillie de verrouillage (31c, 331c) est formée par une nervure annulaire qui entoure totalement et sans interruption la queue (25, 125, 225, 325).

6. Dispositif selon la revendication 4 ou 5, caractérisé en ce que la rainure de verrouillage (25d, 125d, 225d) et la saillie de verrouillage (31c) se trouvent sur le côté du canal (41, 141, 241) opposé à la partie coupante (23, 123, 223).

7. Dispositif selon la revendication 4 ou 5, caractérisé en ce que la rainure de verrouillage (325d) forme une partie de la limitation du canal (341).

8. Dispositif selon l'une des revendications 1 à 7, caractérisé en ce que le canal annulaire (41, 141, 241, 341) est fermé vis-à-vis de l'environnement de l'outil (21, 121, 221, 321) et du manchon (31, 131, 231, 331), tant sur son côté tourné vers la partie coupante (23, 123, 223, 323) que sur son côté opposé à celle-ci, exclusivement par des sections de l'outil (21, 121, 221, 321) et du manchon (31, 131, 231, 331), le manchon (31, 131, 231, 331) comportant par exemple un trou axial (31a) traversant, muni d'un élargissement (31b) à l'extrémité tournée vers la partie coupante, et traversé par l'outil (21, 121, 321), et l'outil (21, 121, 321) comportant par exemple sur le côté du canal annulaire (41, 141, 341) qui est tourné vers la partie coupante (23, 123, 323) un épaulement formé par un renflement (25b) de la queue (25) et/ou par la partie coupante (23, 123) et situé à l'intérieur de l'élargissement (31b).

9. Dispositif selon l'une des revendications 1 à 8, caractérisé en ce que la queue (25, 125, 225, 325) qui porte le manchon (31, 131, 231, 331) est reliée de manière rigide et indétachable à la partie coupante (23, 123, 223, 323) et est par exemple soudée à celle-ci ou constituée avec celle-ci par un corps d'une pièce, le manchon (31, 131, 231, 331) étant constitué judicieusement par une matière plastique déformable élastiquement qui est de préférence moulable par injection et qui de préférence résiste à la la chaleur jusqu'à une température d'au moins 120°C et par exemple d'au moins 180°.

10. Dispositif selon l'une des revendications 1 à 9, dans lequel le passage (43, 343) comporte un trou axial (23c, 323c) avec une ouverture de sortie (45) qui débouche dans l'extrémité libre de la partie coupante (23, 323), caractérisé en ce que la queue (25, 325) présente une entaille axiale (25e, 325e) qui relie le canal annulaire (41, 141) au trou axial (23c, 323c) du passage (41, 341), dont le fond est incliné, au moins dans un domaine partiel, par rapport à la direction longitudinale de l'outil (21, 321) de telle manière que sa profondeur mesurée radialement augmente en direction de l'extrémité libre de la partie coupante, et en ce que l'entaille (25e, 325e) est fermée vis-à-vis de l'environnement par le manchon (31, 331) et/ou la partie coupante (23, 123), la partie coupante (23, 323) et la queue (25, 325) étant de préférence constituées par des pièces usinées séparées à l'origine, la pièce usinée formant la queue (25, 325) faisant de préférence saillie dans la pièce usinée formant la partie coupante (23, 323) et l'entaille (25e, 325e) s'étendant de préférence jusqu'à l'extrémité de la pièce usinée formant la queue (25, 225) située du côté de la partie coupante.
